(19) Europäisches Patentamt
European Patent Office
Office européen des brevets

(11) EP 2 315 576 B1

(12) EUROPÄISCHE PATENTSCHRIFT

(45) Veröffentlichungstag und Bekanntmachung des Hinweises auf die Patenterteilung:
**25.12.2013 Patentblatt 2013/52**

(21) Anmeldenummer: **09779668.4**

(22) Anmeldetag: **08.06.2009**

(51) Int Cl.:
***A61K 8/49*** (2006.01) ***A61Q 5/10*** (2006.01)

(86) Internationale Anmeldenummer:
**PCT/EP2009/057015**

(87) Internationale Veröffentlichungsnummer:
**WO 2010/022996 (04.03.2010 Gazette 2010/09)**

# (54) KATIONISCHE ACETYLPYRIDINIUM-DERIVATE ALS BLEICHAKTIVATOREN

CATIONIC ACETYLPYRIDINIUM DERIVATIVES FOR USE AS BLEACH ACTIVATORS

DÉRIVÉS CATIONIQUES D'ACÉTYLPYRIDINE SERVANT D'ACTIVATEURS D'ÉCLAIRCISSEMENT

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **28.08.2008 DE 102008044715**

(43) Veröffentlichungstag der Anmeldung:
**04.05.2011 Patentblatt 2011/18**

(73) Patentinhaber: **Henkel AG & Co. KGaA**
**40589 Düsseldorf (DE)**

(72) Erfinder:
- **GROß, Wibke**
**41836 Hückelhoven (DE)**
- **FUHR, Denise**
**22559 Hamburg (DE)**
- **NEMITZ, Ralph**
**41363 Jüchen (DE)**
- **KLEEN, Astrid**
**20457 Hamburg (DE)**

(56) Entgegenhaltungen:
**EP-A- 1 800 655**
**EP-A- 1 882 495**
**EP-A- 1 891 927**
**EP-A- 1 905 418**
**WO-A-2005/115322**
**DE-A1- 19 745 356**
**DE-A1-102006 020 789**
**DE-A1-102007 047 685**

Bemerkungen:
Die Akte enthält technische Angaben, die nach dem Eingang der Anmeldung eingereicht wurden und die nicht in dieser Patentschrift enthalten sind.

EP 2 315 576 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft ein Mittel zur Farbveränderung bei gleichzeitiger Aufhellung von keratinhaltigen Fasern, insbesondere menschlichen Haaren welches unmittelbar von der Anwendung durch Vermischen mindestens zweier Zubereitungen hergestellt wird und, welches in einem kosmetischen Träger mindestens eine farbverändernde Verbindung sowie ein kationisches Derivat eines Acylpyridins gemäß Anspruch 1 enthält. Weiterhin betrifft die Erfindung ein Verfahren zur Anwendung von solchen Mitteln auf keratinhaltigen Fasern. Darüber hinaus betrifft die Erfindung die Verwendung des Mittels zur Färbung und gleichzeitigen Aufhellung der Haare.

Die Veränderung von Form und insbesondere von Farbe der Haare stellt einen wichtigen Bereich der modernen Kosmetik dar. Dadurch kann das Erscheinungsbild der Haare sowohl aktuellen Modeströmungen als auch den individuellen Wünschen der einzelnen Person angepasst werden. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und vorzugsweise sogar zusätzliche Pflegeeigenschaften besitzen.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung diverse Färbesysteme.Sollen Substrate aufgehellt oder gar gebleicht werden, wird die ursprüngliche Färbung meist oxidativ unter Einsatz von entsprechenden Oxidationsmitteln, wie beispielsweise Wasserstoffperoxid, entfernt.

Das Färben, insbesondere das aufhellende Färben von dunklem Haar ist ein zentraler Wunsch vieler Verbraucher. Gerade für Konsumenten mit stärker pigmentiertem Haar ist eine ausreichende Aufhellung grundlegende Voraussetzung für die Erzeugung von intensiven Nuancen im Mode- und Naturtonbereich, die heller als die ursprüngliche Ausgangshaarfarbe sind. Durch Anwendung von Wasserstoffperoxid allein lässt sich bei dunkelblondem, braunem oder schwarzem Haar nur schwierig eine signifikante Aufhellung erzielen.

Aus dem Stand der Technik ist zur Verstärkung der Blondierwirkung der Einsatz einer Kombination von Wasserstoffperoxid und Persulfatsalzen bekannt. Diese Oxidationsmittelgemische bewirken jedoch in der Regel sowohl eine starke Schädigung des Haares als auch eine oxidative Zerstörung der im Zuge des Färbeprozesses aus Farbstoffvorprodukten vom Entwickler- und Kuppler-Typ entstehenden Farbstoffe, so dass auf diesem Wege keine gleichzeitige starke Aufhellung und Färbung erreicht werden kann.

Oxidative Haarfärbemittel, insbesondere mit zusätzlicher Blondierwirkung, sind jedoch auch mit weiteren Nachteilen behaftet. Einerseits führt der Einsatz der Oxidationsmittel zu Schädigungen in der Haarstruktur und auf der Haaroberfläche. Das Haar wird brüchig, seine Elastizität lässt nach, und die Kämmbarkeit nimmt ab. Diese Schädigung nimmt mit der Anwendungsdauer zu. Handelsübliche oxidative Färbemittel müssen meist über einen Zeitraum von 30 Minuten und länger auf die Haarfaser einwirken. Die Erhöhung der Einwirkzeit führt zu einer gesteigerten Beeinträchtigung der Haarstruktur. Andererseits benötigen oxidative Färbemittel in der Regel einen basischen pH-Wert zur Ausfärbung, insbesondere zwischen pH 9,0 und pH 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Das basische Milieu stellt jedoch einen weiteren Grund der Schädigung des Haares und dessen Struktur dar, der ebenfalls mit gesteigerter Anwendungszeit an Bedeutung gewinnt. Die Spreizung der äußeren Schuppenschicht führt außerdem zu einem unangenehmen Oberflächenempfinden der Haare und damit zu einer verschlechterten Kämmbarkeit im Nass- und Trockenzustand. Weiterhin wird der basische pH-Wert häufig mit Ammoniak als Alkalisierungsmittel eingestellt, da ammoniakhaltige Färbemittel zusätzliche Vorteile hinsichtlich der Färbeleistung besitzen. Für den Anwender besitzt ein solches Färbemittel jedoch den Nachteil, dass es durch Ammoniak neben zusätzlichen Schädigungen des Haares zu Reizungen von Augen oder Kopfhaut kommen kann, wodurch Sensibilisierungen oder gar allergische Reaktionen hervorgerufen werden können. Darüber hinaus besitzen solche Färbemittel einen intensiven, unangenehmen Geruch, der schlimmstenfalls auch zu Reizungen der Nasenschleimhaut führen kann. Außerdem kann auch die Produktion und Lagerung von Ammoniakhaltigen Färbemitteln mit Problemen hinsichtlich Handhabbarkeit und Stabilität verbunden sein.

DE 10 2007 047 685 A1 beschreibt Mittel zum Aufhellen von keratinischen Fasern, welche kationische Acylpyridiniumderivate, Imidzolderivate und Wasserstoffperoxid enthalten. Die Mittel sollen eine verbesserte Aufhellleistung besitzen.

Aufgabe der vorliegenden Erfindung ist es daher, Färbemittel für die oxidative Färbung von Haaren bereit zu stellen, welche eine maßgebliche Blondierwirkung besitzen, die Haare aber gleichzeitig in intensiven und brillanten Nuancen zu färben vermögen.

Für eine Färbung mit permanenten Haarfarben muss das Farbstoffgemisch in der Regel für einen Zeitraum von 30 bis 45 Minuten auf den Haaren verbleiben. Eine Verkürzung der Einwirkzeit bedeutet für den Konsumenten eine starke Vereinfachung des Haarfärbeprozesses und Steigerung des Komforts während der Anwendung. Obwohl die Verkürzung der Einwirkzeit als wünschenswert eingestuft wird, ist die technische Realisierbarkeit problematisch, da kurze Applikationszeiten ebenfalls in einer schwächeren Aufhellung des Haares resultieren. Aus diesem Grund besteht

die Aufgabe dieser Erfindung darüber hinaus in der Auffindung von oxidativen Färbemitteln mit gegenüber dem Stand der Technik überlegener Aufhellleistung zur Verkürzung der Einwirkzeit. Schließlich ist es wünschenswert, farb- und/ oder formverändernde Mittel bereitzustellen, bei denen das Reizpotential, insbesondere hervorgerufen durch den Zusatz von Ammoniak, möglichst niedrig ist.

Überraschenderweise konnte nun gefunden werden, dass das oben gestellte Anforderungsprofil durch den Einsatz einer Kombination aus Oxidationsfarbstoffvorprodukten sowie speziellen Bleichaktivatoren in hervorragender Weise erfüllt werden kann.

Ein erster Gegenstand der Erfindung ist daher ein Mittel zum Färben und gleichzeitigem Aufhellen von keratinischen Fasern, dadurch gekennzeichnet, dass es unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel (A), welches in einem kosmetischen Träger mindestens ein Oxidationsfarbstoffvorprodukt sowie mindestens ein Acylpyrimidinium-Derivat gemäß der Formel (I) enthält,

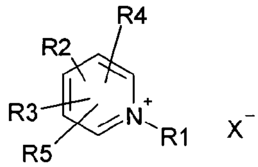

(I),

worin

| | |
|---|---|
| R1 | für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe steht, |
| R2 | für eine Acylgruppe R'C(O) steht, in der R' für eine $C_1$-$C_6$-Alkylgruppe steht, |
| R3, R4 und R5 | unabhängig voneinander ein Wasserstoffatom, eine Hydroxygruppe, eine Aminogruppe, eine Di-($C_1$-$C_6$-alkyl)aminogruppe, eine $C_1$-$C_6$-Alkoxygruppe, eine $C_1$-$C_6$-Alkoxy-$C_2$-$C_6$-alkylgruppe, ein Halogen, eine Nitrogruppe, eine Carboxygruppe, eine Nitrilgruppe, eine gegebenenfalls substituierte Arylgruppe, eine $C_1$-$C_6$-Alkylgruppe, eine $C_2$-$C_6$-Alkenylgruppe, eine gegebenenfalls substituierte Heteroarylgruppe darstellen können, und |

das Anion $X^-$ für ein physiologisch verträgliches Anion steht.

und ein weiterer Container eine Oxidationsmittelzubereitung (B), enthaltend mindestens ein Oxidationsmittel, enthält.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die erfindungsgemäßen Mittel enthalten die Wirkstoffe in einem kosmetischen Träger. Dieser kosmetische Träger ist im Sinne der Erfindung ist wässrig, alkoholisch oder wässrigalkoholisch. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmonon-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung.

Als wesentlichen Inhaltsstoff enthält das erfindungsgemäße Mittel einen kationisches Acylpyridinium-Derivat. Dabei handelt es sich um ein physiologisch verträgliches Salz gemäß Formel (I),

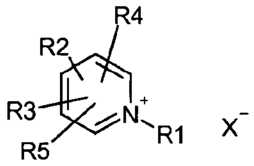

(I),

worin

R1 für eine $C_1$-$C_6$-Alkylgruppe, eine $C_2$-$C_6$-Alkenylgruppe, eine $C_2$-$C_6$-Hydroxy-alkylgruppe, eine $C_1$-$C_6$-Alkoxy-$C_2$-$C_6$-alkylgruppe, eine Carboxy-$C_1$-$C_6$-alkylgruppe, eine Aryl-$C_1$-$C_6$-alkylgruppe, eine Heteroaryl-$C_1$-$C_6$-alkyl-gruppe, eine Arylgruppe oder eine Heteroarylgruppe steht,

R2 für eine Acylgruppe R'C(O) steht, in der R' für eine $C_1$-$C_6$-Alkylgruppe, steht,

R3, R4 und R5 unabhängig voneinander ein Wasserstoffatom, eine Hydroxygruppe, eine Aminogruppe, eine Di-($C_1$-$C_6$-alkyl)aminogruppe, eine $C_1$-$C_6$-Alkylgruppe, eine $C_1$-$C_6$-Alkoxygruppe, eine $C_1$-$C_6$-Alkoxy-$C_2$-$C_6$-alkylgruppe, ein Halogen, eine Nitrogruppe, eine Carboxygruppe, eine Nitrilgruppe, eine gegebenenfalls substituierte Arylgruppe, eine $C_2$-$C_6$-Alkenylgruppe, eine gegebenenfalls substituierte Heteroarylgruppe darstellen können, und

das Anion $X^-$ für ein physiologisch verträgliches Anion steht

Beispiele für der Reste R1, R3, R4, R5 und R' in Formel (I) sind:

- für eine $C_1$-$C_6$-Alkylgruppe: Methyl, Ethyl, Propyl, i-Propyl, n-Butyl, s-Butyl, t-Butyl, i-Butyl, n-Pentyl, neo-Pentyl, insbesondere Methyl, Ethyl und i-Propyl;
- für eine $C_2$-$C_6$-Alkenylgruppe: Ethenyl, Allyl, (Z)-Propen-1-yl, (E)-Propen-1-yl, Propen-2-yl, (E)-Buten-1-yl, (Z)-Buten-1-yl, (E)-But-2-en-1-yl, (Z)-But-2-en-1-yl, But-3-en-1-yl, But-2-en-2-yl, But-2-en-3-yl, Pent-4-en-1-yl, Hex-4-en-1-yl, insbesondere Allyl und But-3-en-1-yl;
- für eine $C_2$-$C_6$-Hydroxyalkylgruppe: $CH_2CH_2OH$, $CH_2CH_2CH_2OH$, $CH_2CH(OH)CH_3$, $CH_2CH_2CH_2CH_2OH$, insbesondere $CH_2CH_2OH$ und $CH_2CH_2CH_2OH$;
- für eine $C_1$-$C_6$-Alkoxygruppe: $OCH_3$, $OCH_2CH_3$, $OCH_2CH_2CH_3$, $OCH(CH_3)_2$, $OCH_2CH_2CH_2CH_3$, $OCH_2CH(CH_3)_2$, $OCH(CH_3)CH_2CH_3$, $OC(CH_3)_3$, insbesondere eine Methoxy- oder eine Ethoxygruppe;
- für eine $C_1$-$C_6$-Alkoxy-$C_2$-$C_6$-alkylgruppe: $CH_2CH_2OCH_3$, $CH_2CH_2CH_2OCH_3$, $CH_2CH_2OCH_2CH_3$, $CH_2CH_2CH_2OCH_2CH_3$, $CH_2CH_2OCH(CH_3)_2$, $CH_2CH_2CH_2OCH(CH_3)_2$;
- für eine Carboxy-$C_1$-$C_6$-alkylgruppe: $CH_2CO_2H$, $CH_2CH_2CO_2H$, $CH_2CH_2CH_2CO_2H$, $CH_2CH(CH_3)CO_2H$ oder eines der physiologisch verträglichen Salze davon;
- für eine Aryl-$C_1$-$C_6$-alkylgruppe: Benzyl, 1-Phenylethyl, 2-Phenylethyl;
- für eine Di-($C_1$-$C_6$-alkyl)amino-gruppe: $N(CH_3)_2$, $N(CH_3)CH_2CH_3$, $N(CH_2CH_3)_2$;
- für eine Heteroaryl-$C_1$-$C_6$-alkylgruppe: (Pyridin-2-yl)methyl, (Pyridin-3-yl)methyl, (Pyridin-4-yl)methyl, (Pyridin-2-yl) ethyl, (Pyrimidin-4-yl)methyl, (Imidazol-1-yl)methyl, (Imidazol-2-yl)methyl, (Imidazol-4-yl)methyl, (Thiazol-4-yl)methyl;
- für eine Heteroarylgruppe: Pyridin-2-yl, Pyridin-3-yl, Pyridin-4-yl, Pyrimidin-4-yl, Imidazol-1-yl, Imidazol-2-yl, Imidazol-4-yl, Thiazol-4-yl, Thiazol-5-yl, Pyrrol-1-yl, Pyrrol-2-yl, Pyrrol-3-yl, Pyrazol-1-yl, Pyrazol-3-yl, Pyrazol-4-yl, 1,2,3-Triazol-1-yl, 1,2,3-Triazol-4-yl;
- für eine Arylgruppe: Phenyl, Naphthalin-1-yl und Naphthalin-2-yl;
- für Halogen: Fluor, Chlor, Brom, Iod, insbesondere Chlor.

Eine besondere Ausführungsform der vorliegenden Erfindung ist dadurch gekennzeichnet, dass der Rest R1 in der Formel (I) für eine $C_1$-$C_6$-Alkylgruppe, für eine $C_2$-$C_6$-Alkenylgruppe oder für eine $C_2$-$C_6$-Hydroxyalkylgruppe steht.

Erfindungsgemäße Verbindungen sind dadurch gekennzeichnet, dass der Rest R2 gemäß Formel (I) für eine Acylgruppe R'C(O) steht, in der R' für eine $C_1$-$C_6$-Alkylgruppe, insbesondere für eine Methylgruppe, steht. Ganz besonders bevorzugte Verbindungen gemäß Formel (I) tragen den Rest R2, welcher für eine Acylgruppe R'C(O) steht, in der 4-bzw. in der 2-Position. Diese besonders bevorzugten Ausführungsformen der Verbindung gemäß Formel (I) sind explizit durch die Formeln (Ia) bzw. (Ib) offenbart:

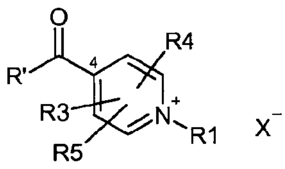 (Ia) bzw. 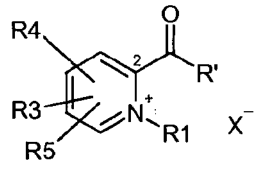 (Ib)

Die Reste R1, R3, R4, R5 und R' sind wie oben beschrieben definiert. ,

Weiterhin kann es erfindungsgemäß vorteilhaft ein, wenn die Reste R3, R4 und R5 in Formel (I) jeweils ein Wasserstoffatom stehen.

Es ist bevorzugt, wenn X- gemäß Formel (I) ausgewählt wird aus Halogenid (Chlorid, Bromid, Iodid), Benzolsulfonat, p-Toluolsulfonat, $C_1$-$C_4$-Alkansulfonat, Trifluormethansulfonat, Acetat, Trifluoracetat, Perchlorat, ½ Sulfat, Hydrogensulfat, Tetrafluoroborat, Hexafluorophosphat, Hexafluorozinkat oder Tetrafluorozinkat.

Erfindungsgemäß besonders begünstigt ist es, wenn das physiologisch verträgliche Anion Xfür ein Halogenidion (insbesondere Chlorid oder Bromid), Hydrogensulfat, ½ Sulfat, p-Toluolsulfonat, Benzolsulfonat oder Acetat steht.

Besonders bevorzugte kationische Acylpyridinium-Derivate der allgemeinen Formel (I) sind

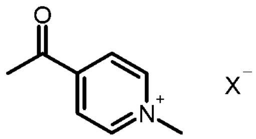

Salze des 4-Acetyl-1-methylpyridiniums;

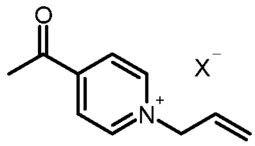

Salze des 4-Acetyl-1-allylpyridiniums;

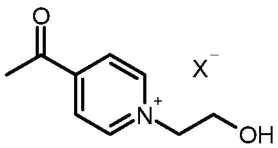

Salze des 4-Acetyl-1-(2-hydroxyethyl)pyridiniums;

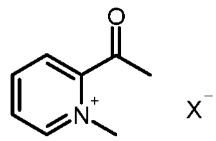

Salze des 2-Acetyl-1-methylpyridiniums;

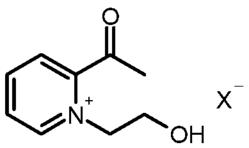

Salze des 2-Acetyl-1-(2-hydroxyethyl)pyridiniums;

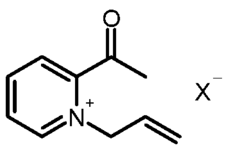

Salze des 2-Acetyl-1-allylpyridiniums;

worin $X^-$ jeweils die Bedeutungen gemäß Struktur (I), bzw. die Bedeutung der vorgenannten bevorzugten Ausführungsformen, annimmt.

Insbesondere sind solche Mittel ganz besonders bevorzugt, die dadurch gekennzeichnet sind, dass mindestens ein Acylpyridinium-Derivat gemäß Formel (I) ausgewählt aus Verbindungen der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methyl-pyridiniumchlorid, 4-Acetyl-1-methylpyridiniumhydrogensulfat, 4-Acetyl-1-methylpyridinium-acetat, 4-Acetyl-1-allylpyridinium-p-toluolsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridinium-hydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methyl-pyridiniumacetat, 2-Acetyl-1-allylpyridinium-p-toluolsulfonat, 2-Acetyl-1-allylpyridinium-benzol-sulfonat, 2-Acetyl-1-allylpyridiniumbromid, 2-Acetyl-1-allylpyridiniumchlorid, 2-Acetyl-1-allyl-pyridiniumhydrogensulfat oder 2-Acetyl-1-allylpyridiniumacetat, enthalten ist.

Ganz besonders bevorzugt enthalten die erfindungsgemäßen Mittel als Acylpyridinium-Derivat 2-Acetyl-1-methylpyridinium-p-toluolsulfonat und/oder 4-Acetyl-1-methylpyridinium-p-toluolsulfonat.

Als wesentlichen Inhaltstoff enthalten die erfindungsgemäßen Mittel die Acylpyridinium-Derivate gemäß Formel (I) zu 0,01 bis 15 Gew.-%, bevorzugt zu 0,1 bis 12 Gew.-% und ganz besonders bevorzugt zu 0,5 bis 5 Gew.-%, jeweils bezogen auf das Gesamtgewicht des anwendungsbereiten Mittels.

Als weiteren wesentlichen Inhaltsstoff enthalten die erfindungsgemäßen Mittel mindestens ein Oxidationsfarbstoffvorprodukt. Oxidationsfarbstoffvorprodukte werden aufgrund ihres mechanistischen Verhaltens während der eigentlichen Farbstoffausbildung in Vorprodukte vom Entwicklertyp und vom Kupplertyp unterteilt. In einer bevorzugten Ausführungsform enthalten die erfindungsgemäßen Mittel mindestens ein Oxidationsfarbstoffvorprodukt vom Entwicklertyp und/oder Kupplertyp. Vorzugsweise enthalten die erfindungsgemäßen Färbemittel mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Bevorzugte p-Phenylendiamine werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Di-ethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylen-diamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylen-diamin, 2-(2-Acetylaminoethyloxy)-p-phenylendiamin, N-(2-Methoxyethyl)-p-phenylendiamin, N-(4-Amino-3-methylphenyl)-N-[3-(1 H-imidazol-1-yl)propyl]amin, 5,8-Diaminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere aus mindestens einer der folgenden Verbindungen ausgewählt: N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methyl-amino)phenyl)tetramethylendiamin, N,N'-Diethyl-N,N'-bis-(4'-amino-3'-methylphenyl)-ethylendiamin, Bis-(2-hydroxy-5-aminophenyl)methan, N,N'-Bis-(4'-aminophenyl)-1,4-diaza-cycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diaminopropan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)phenol, 4-Amino-2-methylphenol, 4-Amino-2-hydroxymethylphenol, 4-Amino-2-methoxymethylphenol, 4-Amino-2-aminomethyl-phenol, 4-Amino-2-(2-hydroxyethylaminomethyl)phenol, 4-Amino-2-(1,2-dihydroxyethyl)-phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugt sind p-Aminophenol, 4-Amino-3-methylphenol, 4-Amino-2-aminomethylphenol, 4-Amino-2-(1,2-dihydroxyethyl) phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Bevorzugte PyrimidinDerivate werden erfindungsgemäß ausgewählt aus den Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate werden erfindungsgemäß ausgewählt aus 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Bevorzugte Pyrazolopyrimidin-Derivate sind insbesondere ausgewählt aus Pyrazolo[1,5-a]pyrimidin-3,7-diamin; 2,5-Di-methylpyrazolo [1,5-a]pyrimidin-3,7-diamin; Pyrazolo[1,5-a]pyrimidin-3,5-diamin; 2,7-Dimethyl-pyrazolo[1,5-a]pyrimidin-3,5-diamin; 3-Aminopyrazolo[1,5-a]pyrimidin-7-ol; 3-Aminopyrazolo-[1,5-a]pyrimidin-5-ol; 2-(3-Aminopyrazolo[1,5-a]pyrimidin-7-ylamino)ethanol; 2-(7-Amino-pyrazolo[1,5-a]pyrimidin-3-ylamino)ethanol; 2-[(3-Aminopyrazolo[1,5-a]pyrimidin-7-yl)-(2-hydroxyethyl)amino]ethanol; 2-[(7-Aminopyrazolo[1,5-a]pyrimidin-3-yl)-(2-hydroxyethyl)-amino]ethanol; 5,6-Dimethylpyrazolo[1,5-a]pyrimidin-3,7-diamin; 2,6-Dimethylpyrazolo[1,5-a]-pyrimidin-3,7-diamin; 3-Amino-7-dimethylamino-2,5-dimethylpyrazolo[1,5-a]pyrimidin wie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methyl-phenyl)-N-[3-(1 H-imidazol-1-yl)propyl]amin, N,N'-Bis-(2-hydroxyethyl)-N,N'-bis-(4-amino-phenyl)-1,3-diamino-propan-2-ol, Bis-(2-hydroxy-5-aminophenyl)methan, 1,3-Bis-(2,5-di-aminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Ganz besonders bevorzugte Entwicklerkomponenten sind dabei p-Toluylendiamin, 2-(2-Hydroxyethyl)-p-phenylendiamin, 2-Methoxy-methyl-p-phenylendiamin, N-(4-Amino-3-methylphenyl)-N-[3-(1 H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Bevorzugte m-Aminophenol-Kupplerkomponenten werden ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxy-ethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und den physiologisch verträglichen Salzen aller vorstehend genannten Verbindungen.

Bevorzugte m-Diaminobenzol-Kupplerkomponenten werden ausgewählt aus mindestens einer Verbindung aus der Gruppe, die gebildet wird aus m-Phenylendiamin, 2-(2,4-Diamino-phenoxy)ethanol, 1,3-Bis(2,4-diaminophenoxy) propan, 1-Methoxy-2-amino-4-(2'-hydroxy-ethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)-amino]-4,5-dimethylphenyl}amino) ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und den physiologisch verträglichen Salzen aller vorstehend genannten Verbindungen.

Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Di-amino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxy-naphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen. Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-amino-phenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)-ethanol, 1,3-Bis(2,4-diaminophenoxy)propan, 1-Methoxy-2-amino-4-(2-hydroxyethylamino)-benzol, 1,3-Bis(2,4-diaminophenyl)propan, 2,6-Bis(2'-hydroxyethylamino)-1-methylbenzol, 2-({3-[(2-Hydroxyethyl) amino]-4-methoxy-5-methylphenyl}amino)ethanol, 2-({3-[(2-Hydroxy-ethyl)amino]-2-methoxy-5-methylphenyl}amino) ethanol, 2-({3-[(2-Hydroxyethyl)amino]-4,5-di-methylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen. Ganz besonders bevorzugt ist dabei Resorcin, 2-Methylresorcin, 5-Amino-2-methylphenol, 3-Aminophenol, 2-(2,4-Diaminophenoxy)ethanol, 1,3-Bis (2,4-di-aminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Im Rahmen der vorliegenden Erfindung sind folgende Kombinationen aus Oxidationsfarbstoffvorprodukten vom Entwicklertyp und vom Kupplertyp besonders bevorzugt. Mit den als Kombination genannten Oxidationsfarbstoffvorprodukten können jedoch auch noch weitere Farbstoffvorprodukte kombiniert werden: p-Toluylendiamin / Resorcin; p-Toluylendiamin / 2-Methylresorcin; p-Toluylendiamin / 5-Amino-2-methylphenol; p-Toluylendiamin / 3-Amino-phenol; p-Toluylendiamin / 2-(2,4-Diaminophenoxy)ethanol; p-Toluylendiamin / 1,3-Bis(2,4-diaminophenoxy)propan; p-Toluylendiamin / 1-Methoxy-2-amino-4-(2-hydroxyethylamino)-benzol; p-Toluylendiamin / 2-Amino-3-hydroxypyridin; p-Toluylendiamin / 1-Naphthol; 2-(2-Hydroxyethyl)-p-phenylendiamin / Resorcin; 2-(2-Hydroxyethyl)-p-phenylendiamin / 2-Methylresorcin; 2-(2-Hydroxyethyl)-p-phenylendiamin / 5-Amino-2-methylphenol; 2-(2-Hydroxyethyl)-p-phenylendiamin / 3-Aminophenol; 2-(2-Hydroxyethyl)-p-phenylendiamin / 2-(2,4-Diamino-phenoxy)ethanol; 2-(2-Hydroxyethyl)-p-phenylendiamin / 1,3-Bis(2,4-diaminophenoxy)propan; 2-(2-Hydroxyethyl)-p-phenylendiamin / 1-Methoxy-2-amino-4-(2-hydroxyethylamino)benzol; 2-(2-Hydroxyethyl)-p-phenylendiamin / 2-Amino-3-hydroxypyridin; 2-(2-Hydroxyethyl)-p-phenylendiamin / 1-Naphthol; 2-Methoxymethyl-p-phenylendiamin / Resorcin; 2-Methoxymethyl-p-phenylendiamin / 2-Methylresorcin; 2-Methoxymethyl-p-phenylendiamin / 5-Amino-2-methylphenol; 2-Methoxymethyl-p-phenylendiamin / 3-Aminophenol; 2-Methoxymethyl-p-phenylendiamin / 2-(2,4-Diaminophenoxy)ethanol; 2-Methoxymethyl-p-phenylendiamin / 1,3-Bis(2,4-diaminophenoxy)propan; 2-Methoxymethyl-p-phenylendiamin / 1-Methoxy-2-amino-4-(2-hydroxyethylamino)benzol; 2-Methoxymethyl-p-phenylendiamin / 2-Amino-3-hydroxy-pyridin; 2-Methoxymethyl-p-phenylendiamin / 1-Naphthol; N-(4-Amino-3-methylphenyl)-N-[3-(1H-imidazol-1-yl)propyl]amin / Resorcin; N-(4-Amino-3-methylphenyl)-N-[3-(1H-imidazol-1-yl)propyl]amin / 2-Methylresorcin; N-(4-Amino-3-methylphenyl)-N-[3-(1 H-imidazol-1-yl)propyl]-amin / 5-Amino-2-methylphenol; N-(4-Amino-3-methylphenyl)-N-[3-(1H-imidazol-1-yl)propyl]-amin / 3-Aminophenol; N-(4-Amino-3-methylphenyl)-N-[3-(1H-imidazol-1-yl)propyl]amin / 2-(2,4-Diaminophenoxy)ethanol; N-(4-Amino-3-methylphenyl)-N-[3-(1 H-imidazol-1-yl)propyl]-amin / 1,3-Bis(2,4-diaminophenoxy)propan; N-(4-Amino-3-methylphenyl)-N-[3-(1H-imidazol-1-yl)propyl]amin / 1-Methoxy-2-amino-4-(2-hydroxyethylamino)benzol; N-(4-Amino-3-methylphenyl)-N-[3-(1 H-imidazol-1-yl)propyl]amin / 2-Amino-3-hydroxypyridin; N-(4-Amino-3-methylphenyl)-N-[3-(1H-imidazol-1-yl)propyl]amin / 1-Naphthol; 4,5-Diamino-1-(2-hydroxyethyl)-pyrazol / Resorcin; 4,5-Diamino-1-(2-hydroxyethyl) pyrazol / 2-Methylresorcin; 4,5-Diamino-1-(2-hydroxyethyl)pyrazol / 5-Amino-2-methylphenol; 4,5-Diamino-1-(2-hydroxyethyl)pyrazol / 3-Aminophenol; 4,5-Diamino-1-(2-hydroxyethyl)pyrazol / 2-(2,4-Diaminophenoxy)ethanol; 4,5-Diamino-1-(2-hydroxyethyl)pyrazol / 1,3-Bis(2,4-diaminophenoxy)propan; 4,5-Diamino-1-(2-hydroxyethyl)pyrazol / 1-Methoxy-2-amino-4-(2-hydroxyethylamino)benzol; 4,5-Diamino-1-(2-hydroxyethyl)pyrazol / 2-Amino-3-hydroxypyridin; 4,5-Diamino-1-(2-hydroxyethyl)pyrazol / 1-Naphthol.

Ganz besonders vorteilhaft ist es im Rahmen der vorliegenden Erfindung, wenn die erfindungsgemäßen Mittel mindestens eine der voranstehenden Oxidationsfarbstoffvorprodukt-Kombinationen zusammen mit 4-Acetyl-1-methylpyridinium-p-toluolsulfonat als Acylpyridinium-Derivat enthalten. Ebenso ist es im Rahmen der vorliegenden Erfindung ganz besonders vorteilhaft, wenn die erfindungsgemäßen Mittel mindestens eine der voranstehenden Oxidationsfarbstoffvorprodukt-Kombinationen zusammen mit 2-Acetyl-1-methylpyridinium-p-toluolsulfonat als Acylpyridinium-Derivat enthalten.

Die Oxidationsfarbstoffvorprodukte werden im Rahmen der vorliegenden Erfindung bevorzugt in einer Menge von 0,005 bis 20 Gew.-%, bevorzugt von 0,05 bis 5 Gew.-% und besonders bevorzugt von 0,1 bis 5 Gew.-%, jeweils bezogen auf das anwendungsbereite Oxidationsfärbemittel, verwendet. Besonders bevorzugt ist es im Rahmen der vorliegenden Erfindung, wenn die Kombinationen aller Oxidationsfarbstoffvorprodukte und der Acylpyridinium-Derivate gemäß Formel (I) in einer Menge von 0,05 bis 20 Gew.-%, bevorzugt von 0,1 bis 15 Gew.-% und besonders bevorzugt von 0,5 bis 12 Gew.-%, jeweils bezogen auf das anwendungsbereite Oxidationsfärbemittel, eingesetzt werden. Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Das erfindungsgemäße Mittel kann in einer weiteren Ausführungsform neben den Oxidationsfarbstoffvorprodukten noch mindestens eine weitere farbgebende Komponente enthalten. Die weitere farbgebende Komponente wird bevorzugt ausgewählt aus mindestens einem direktziehenden Farbstoff. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 20 Gew.-%, bezogen auf die gesamte Anwendungszubereitung, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind dabei kationische Triphenylmethanfarbstoffe, wie beispielsweise Basic Blue 7, Basic Blue 26, Basic Violet 2 und Basic Violet 14, aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie direktziehende Farbstoffe, die einen Heterocyclus enthalten, der mindestens ein quaternäres Stickstoffatom aufweist. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureido-ethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass Oxidationsfarbstoffvorprodukte oder direktziehende Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bei der oxidativen Färbungen wird ein chemisches Oxidationsmittel eingesetzt. Als Oxidationsmittel kommen Persulfate, Peroxodisulfate, Chlorite, Hypochlorite und insbesondere Wasserstoffperoxid oder und/oder eines seiner festen Anlagerungsprodukte an organische oder anorganische Verbindungen in Frage. Um eine vorzeitige, unerwünschte Reaktion der Oxidationsfarbstoffvorprodukte durch das Oxidationsmittel zu verhindern, werden Oxidationsfarbstoffvorprodukte und Oxidationsmittel selbst zweckmäßigerweise getrennt voneinander konfektioniert und erst unmittelbar vor der Anwendung in Kontakt gebracht.

Ein Gegenstand der vorliegenden Erfindung ist daher ein Mittel, welches dadurch gekennzeichnet ist, dass es unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden und wobei ein Container ein Färbemittel (A), welches in einem kosmetischen Träger mindestens ein Oxidationsfarbstoffvorprodukt

sowie mindestens ein Acylpyridinium-Derivat gemäß der Formel (I) enthält, und ein weiterer Container eine Oxidationsmittelzubereitung (B), enthaltend mindestens ein Oxidationsmittel, enthält.

Bevorzugt enthält die Oxidationsmittelzubereitung (B) als Oxidationsmittel Wasserstoffperoxid und/oder eines seiner festen Anlagerungsprodukte an organische oder anorganische Verbindungen, wie Harnstoff, Melamin sowie Natriumborat. Bevorzugt beträgt die Menge an Oxidationsmittel im anwendungsbereiten Mittel 0,5 bis 12 Gew.-%, bevorzugt 2 bis 10 Gew.-% insbesondere bevorzugt zu 3 bis 6 Gew.-% (berechnet als 100 %-iges $H_2O_2$), jeweils bezogen auf das anwendungsbereite Mittel.

Solche Oxidationsmittelzubereitungen sind vorzugsweise wässrige, fließfähige Oxidationsmittelzubereitungen. Dabei sind bevorzugte Zubereitungen dadurch gekennzeichnet, dass die fließfähige Oxidationsmittelzubereitung - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen. Geeignete Enzyme sind z. B. Peroxidasen, die die Wirkung geringer Mengen an Wasserstoffperoxid deutlich verstärken können. Ein Einsatz bestimmter Metallionen oder -komplexe kann ebenfalls bevorzugt sein, um intensive, langanhaltende Färbungen zu erhalten. Geeignete Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$, $Ce^{4+}$, $V^{3+}$, $Co^{2+}$, $Ru^{3+}$ und $Al^{3+}$.

Weiterhin hat es sich als vorteilhaft erweisen, wenn die Oxidationsmittelzubereitungen (b) mindestens einen Stabilisator oder Komplexbildner enthalten. Besonders bevorzugte Stabilisatoren sind Phenacetin, Alkalibenzoate (Natriumbenzoat) und Salicylsäure. Erfindungsgemäß bevorzugt ist auch der Einsatz von sogenannten Komplexbildnern. Erfindungsgemäß bevorzugte Komplexbildner sind stickstoffhaltigen Polycarbonsäuren, insbesondere EDTA, und Phosphonate, vorzugsweise Hydroxyalkan- bzw. Aminoalkanphosphonate und insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Die Oxidationsmittelzubereitung enthält neben dem eigentlichen Oxidationsmittel weitere Hilfs- und Zusatzstoffe. So hat es sich erfindungsgemäß als bevorzugt erwiesen, wenn die Oxidationsmittelzubereitung mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Zur weiteren Steigerung der Aufhellleistung können der erfindungsgemäßen Zusammensetzung zusätzlich mindestens eine gegebenenfalls hydratisierte $SiO_2$-Verbindun zugesetzt. Obwohl bereits geringe Mengen der gegebenenfalls hydratisierten $SiO_2$-Verbindungen die Aufhellleistung erhöhen, kann es erfindungsgemäß bevorzugt sein, die gegebenenfalls hydratisierten $SiO_2$-Verbindunge in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Hinsichtlich der gegebenenfalls hydratisierten $SiO_2$-Verbindungen unterliegt die vorliegende Erfindung prinzipiell keinen Beschränkungen. Bevorzugt sind Kieselsäuren, deren Oligomeren und Polymeren sowie deren Salze. Erfindungsgemäß bevorzugt werden die $SiO_2$-Verbindunge in Form von Kieselgelen (Silicagel) oder besonders bevorzugt als Wasserglas eingesetzt. Erfindungsgemäß besonders bevorzugte Wassergläser werden unter anderem von der Firma Henkel unter den Bezeichnungen Ferrosil® 119, Natronwasserglas 40/42, Portil® A, Portil® AW und Portil® W und von der Firma Akzo unter der Bezeichnung Britesil® C20 vertrieben.

Vorzugsweise sind die Oxidationsmittelzubereitung (B) und/oder die Zubereitung (A) als fließfähigen Zubereitungen konfektioniert. Vorzugsweise wird den fließfähigen Zubereitungen (A) und/oder (B) weiterhin ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, amphoteren und nichtionischen Tensiden und Emulgatoren ausgewählt sind. Diese Stoffe werden nachfolgend ausführlich beschrieben.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül.

Besonders geeignete zwitterionische Tenside sind die sogenannten Betaine wie die N-Alkyl-N,N-dimethylammonium-glycinate, , N-Acyl-aminopropyl-N,N-dimethylammoniumglycinate, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind das N-Kokosalkylaminopropionat, das Kokosacylaminoethylaminopropionat und

das $C_{12}$-$C_{18}$-Acylsarcosin.

Weiterhin hat es sich als vorteilhaft erwiesen, wenn die erfindungsgemäßen Färbe- und Aufhellmittel weitere, nichtionogene grenzflächenaktive Stoffe, enthalten. Als nichtionische Tenside eignen sich insbesondere $C_8$-$C_{22}$-Alkylmono- und oligoglycoside und deren ethoxylierte Analoga. Insbesondere die nichtethoxylierten Verbindungen haben sich als geeignet erwiesen. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die anionischen, nichtionischen, zwitterionischen oder amphoteren Tenside werden in Mengen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Weitere erfindungsgemäß verwendbare kationische Tenside stellen die quaternisierten Proteinhydrolysate dar. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar. Ebenfalls sehr gut biologisch abbaubar sind quaternäre Esterverbindungen, sogenannte "Esterquats". Solche Produkte werden beispielsweise unter den Warenzeichen Stepantex®, Dehyquart® und Armocare® vertrieben. Die kationischen Tenside sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

In einer bevorzugten Ausführungsform können nicht-ionische, zwitterionische und/oder amphotere Tenside sowie deren Mischungen bevorzugt sein.

In einer weiteren bevorzugten Ausführungsform kann die Wirkung des erfindungsgemäßen Wirkstoffes durch Emulgatoren gesteigert werden. Solche Emulgatoren sind beispielsweise Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; $C_{12}$-$C_{22}$-Fettsäuremono- und -diester von Anlagerungsprodukten von 1 bis 30 Mol Ethylenoxid an Polyole mit 3 bis 6 Kohlenstoffatomen, insbesondere an Glycerin; $C_8$-$C_{22}$-Alkylmono- und -oligoglycoside; Gemische aus Alkyl-(oligo)-glucosiden und Fettalkoholen; Anlagerungsprodukte von 5 bis 60 Mol Ethylenoxid an Rizinusöl und gehärtetes Rizinusöl; Partialester von Polyolen mit 3 bis 6 Kohlenstoffatomen mit gesättigten Fettsäuren mit 8 bis 22 C-Atomen; Sterine (Zoosterine, Phytosterine, Mykosterine); Phospholipide; Fettsäureester von Zuckern und Zuckeralkoholen, wie Sorbit; Polyglycerine und Polyglycerinderivate; sowie lineare und verzweigte Fettsäuren mit 8 bis 30 C-Atomen und deren Na-, K-, Ammonium-, Ca-, Mg- und Zn-Salze.

Die erfindungsgemäßen Mittel enthalten die Emulgatoren bevorzugt in Mengen von 0,1 bis 25 Gew.-%, insbesondere 0,5 bis 15 Gew.-%, bezogen auf die Gesamtmenge des anwendungsbereiten Mittel.

Bevorzugt können die erfindungsgemäßen Zusammensetzungen mindestens einen nichtionogenen Emulgator bzw. Tensid mit einem HLB-Wert von 8 bis 18, gemäß den im Römpp-Lexikon Chemie (Hrg. J. Falbe, M. Regitz), 10. Auflage, Georg Thieme Verlag Stuttgart, New York, (1997), Seite 1764, aufgeführten Definitionen enthalten. Nichtionogene Emulgatoren bzw. Tenside mit einem HLB-Wert von 10-15 können erfindungsgemäß besonders bevorzugt sein.

Ferner können die erfindungsgemäßen Mittel weitere Wirk-, Hilfs- und Zusatzstoffe enthalten, wie beispielsweise nichtionische Polymere; Silikone wie Polyalkylsiloxane (wie Dimethicone oder Cyclomethicone), Polyarylsiloxane und/oder Polyalkylarylsiloxane; kationische Polymere; zwitterionische und amphotere Polymere; anionische Polymere wie Polyacrylsäuren; Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol; Strukturanten wie Glucose, Maleinsäure und Milchsäure; haarkonditionierende Verbindungen wie Phospholipide, beispielsweise Sojalecithin, Ei-Lecitin und Kephaline sowie Silikonöle; Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Entschäumer wie Silikone; Farbstoffe zum Anfärben des Mittels; Antischuppenwirkstoffe; Aminosäuren und Oligopeptide, insbesondere Arginin und/oder Serin; Lichtschutzmittel; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole;

Ceramide, bevorzugt Sphingolipide oder Pseudoceramide; Vitamine, Provitamine und Vitaminvorstufen, insbesondere solche der Gruppen A, $B_3$, $B_5$, $B_6$, C, E, F und H; Pflanzenextrakte wie die Extrakte aus Grünem Tee, Eichenrinde, Brennnessel, Hamamelis, Hopfen, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Litchi, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel; Cholesterin; Konsistenzgeber wie Zuckerester, Polyolester oder Polyolalkylether; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol /Acrylamid-Copolymere; Perlglanzmittel; Pigmente; Treibmittel wie Propan-Butan-Gemische, $N_2O$, Dimethylether, $CO_2$ und Luft; sowie Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen. Die zusätzlichen Wirk- und Hilfsstoffe werden in den erfindungsgemäßen Mitteln bevorzugt in Mengen von jeweils 0,0001 bis 10 Gew.-%, insbesondere von 0,0005 bis 5 Gew.-%, bezogen auf das Gesamtgewicht der Anwendungsmischung, eingesetzt.

Um die Aufhellwirkung weiter zu verbessern, kann es erfindungsgemäß vorteilhaft sein, wenn die erfindungsgemäßen Mittel zusätzlich einen toxikologisch unbedenklichen Co-Bleichaktivator und/oder dessen physiologisch verträgliches Salz enthalten. Unter dem Begriff "toxikologisch unbedenklich" sind diejenigen Verbindungen zu verstehen, welche keine akute Toxizität, subakute Toxizität und chronische Toxizität sowie insbesondere keine cancerogene, genotoxische oder teratogene Wirkung besitzen. Als toxikologisch unbedenklicher Co-Bleichaktivator im Sinne der vorliegenden Erfindung ist insbesondere nicht Imidazol zu verstehen. Dieser toxikologisch unbedenkliche Co-Bleichaktivator ist bevorzugt ausgewählt aus aliphatischen und/oder carbocyclischen Co-Bleichaktivatoren.

Besonders bevorzugt enthält dieser toxikologisch unbedenkliche Co-Bleichaktivator als wesentliches Strukturmerkmal eine Hydroxygruppe, eine Carbonsäure, einen Schwefelsäuremonoester, einen Phosphorsäuremonoester und/oder ein physiologisch verträgliches Salz davon.

Für den Fall, dass der toxikologisch unbedenkliche Co-Bleichaktivator eine Struktureinheit enthält, die mehrere räumliche Anordnungen erlaubt, wie beispielsweise substituierte Doppelbindungen oder Asymmetriezentren, so sind im Rahmen der vorliegenden Erfindung selbstverständlich alle möglichen Stereoisomeren umfasst. Gegebenfalls kann es aber erfindungsgemäß bevorzugt sein, entweder nur ein mögliches Stereoisomer oder aber explizit ein Gemisch aus zwei oder mehreren Stereoisomeren einzusetzen.

Erfindungsgemäß bevorzugte Mittel sind dadurch gekennzeichnet, dass als Co-Bleichaktivator und/oder dessen physiologisch verträgliches Salz mindestens einen Co-Bleichaktivator gemäß Formel (II) und/oder dessen physiologisch verträgliches Salz enthalten ist,

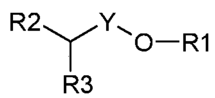

(II)

worin

Y für eine Carbonyl-Gruppe, eine direkte Bindung oder Methylen-Gruppe steht,
R1 für Wasserstoff, eine $C_1$-$C_4$-Alkylgruppe, ein physiologisch verträgliches Kation oder eine $SO_3^-$ oder eine $PO_3^{2-}$-Gruppe steht,
R2 für eine Amino-, eine Methylamino-, eine Dimethylamino-, eine Trimethylammonio-Gruppe, Phenyl, Benzyl, Phenoxymethyl, 1-Naphthyl, 2-Naphthyl, 2-, 3-, 4-Toluolyl, oder eine R4-O-$(CH_2CH_2O)_n$-Gruppe steht, worin R4 für eine $C_6$-$C_{20}$-Alkylgruppe und n für eine Zahl von größer als 15 steht,
R3 für Wasserstoff oder eine gegebenenfalls verzweigte $C_1$-$C_6$-Alkylgruppe steht,
mit der Massgabe, dass,
wenn Y für eine Carbonyl-Gruppe steht,
R1 für Wasserstoff, eine $C_1$-$C_4$-Alkylgruppe oder ein physiologisch verträgliches Kation steht,
R2 für eine Amino-, eine Methylamino-, eine Dimethylamino- oder eine Trimethylammonio-Gruppe steht, und
R3 für Wasserstoff oder eine gegebenenfalls verzweigte $C_1$-$C_6$-Alkylgruppe steht,
dass,
wenn Y für eine direkte Bindung steht,
R1 für Wasserstoff und

R2 für Phenyl, Benzyl, Phenoxymethyl, 1-Naphthyl, 2-Naphthyl, 2-, 3- oder 4-Toluolyl steht, und
R3 für Wasserstoff oder eine gegebenenfalls verzweigte $C_1$-$C_6$-Alkylgruppe steht,
und dass,
wenn Y für eine Methylen-Gruppe steht,
R1 für eine $SO_3^-$- oder eine $PO_3^{2-}$-Gruppe steht,
R2 für eine R4-O($CH_2CH_2O$),-Gruppe steht, worin R4 für eine $C_6$-$C_{20}$-Alkylgruppe und n für eine Zahl von größer als 15 steht, und
R3 für Wasserstoff steht.

Insbesondere sind erfindungsgemäß bevorzugte Mittel dadurch gekennzeichnet, dass als Co-Bleichaktivator mindestens eine gegebenenfalls an ihren Stickstoffatom N-methylierte oder N,N-dimethylierte, aliphatische Aminosäure und/oder dessen physiologisch verträgliches Salz enthalten ist. Bevorzugte Co-Bleichaktivatoren sind ausgewählt aus Glycin, N-Methyl-glycin, N,N-Dimethyl-glycin, Alanin, N-Methyl-alanin, N,N-Dimethyl-alanin, Leucin, N-Methyl-leucin, N, N-Dimethyl-leucin, Isoleucin, N-Methyl-isoleucin, N,N-Dimethyl-isoleucin oder deren physiologisch verträglichen Salzen. Ganz besonders bevorzugt ist im erfindungsgemäßen Mittel als Co-Bleichaktivator Glycin und/oder dessen physiologisch verträgliches Salz enthalten.

Bevorzugte erfindungsgemäße Mittel enthalten als Co-Bleichaktivator mindestens einen aromatischen Alkohol und/oder dessen physiologisch verträgliches Salz. Als erfindungsgemäß bevorzugte, aromatische Alkohole sind Benzylalkohol, 2-Phenylethylalkohol, 1-Phenylethylalkohol, 2-Phenoxyethanol, 1-Hydroxymethyl-naphthalin und/oder 2-Hydroxymethylnaphthalin zu nennen. Ein erfindungsgemäß ganz besonders bevorzugter aromatischer Alkohol als Co-Bleichaktivator ist Benzylalkohol.

Schließlich können solche Mittel erfindungsgemäß bevorzugt sein, die als Co-Bleichaktivator ein physiologisch verträgliches Salz eines Alkylethersulfats gemäß Formel (III)

$$R4\text{-}O(CH_2CH_2O)_mSO_3Y \qquad (III)$$

enthalten, worin R4 für eine $C_6$-$C_{20}$-Alkylgruppe und m für eine Zahl von größer als 15 steht und Y für ein Alkali- und/ oder Erdalkalimetall, Ammonium, Alkylammonium oder Alkanolammonium steht.

Alkylethersulfate ("Ethersulfate") werden großtechnisch durch $SO_3^-$ oder Chlorsulfonsäure (CSA)-Sulfatierung von Fettalkohol- oder Oxoalkoholpolyglycolethern und nachfolgende Neutralisation hergestellt. Erfindungsgemäß bevorzugte Beispiele sind die Sulfate in Form ihrer Natrium- und/oder Magnesiumsalze von hoch ethoxylierten Anlagerungsprodukten von mindestens 16, durchschnittlich jedoch 20 bis 40 und insbesondere 25 bis 35 Mol Ethylenoxid (ausgedrückt durch m in Formel (III)) an Capronalkohol, Caprylalkohol, 2-Ethylhexylalkohol, Caprinalkohol, Laurylalkohol, Isotridecylalkohol, Myristylalkohol, Cetylalkohol, Stearylalkohol, Isostearylalkohol, Eicosylalkohol oder deren technische Mischungen. Diese fallen beispielsweise bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen an. Bevorzugt sind technische Fettalkohole mit 12 bis 18 Kohlenstoftatomen, wie beispielsweise Kokos-, Palm-, Palmkern- oder Talgfettalkohol. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 25 bis 35 Mol Ethylenoxid an technische $C_{12/14}$- bzw. $C_{12/18}$-Kokosfettalkoholfraktionen in Form ihrer Natrium- und/oder Magnesiumsalze. Ein besonders bevorzugter Co-Bleichaktivator ist unter der INCI-Bezeichnung Sodium Coceth-30 sulfate geführt und wird unter dem Handelsnamen Disponil® FES 77 als 31-33 gew.-%ige, wässrige Lösung von der Firma Cognis vertrieben.

Vorzugsweise wird/werden der bzw. die Co-Bleichaktivator(en) innerhalb bestimmter Mengenbereiche eingesetzt. Es sind erfindungsgemäße Mittel bevorzugt, die 0,01 bis 10 Gew.-% und insbesondere 0,1 bis 5 Gew.-%, jeweils bezogen auf das Gesamtgewicht des anwendungsbereiten Mittels, mindestens eines toxikologisch unbedenklichen Co-Bleichaktivators enthalten.

Wird eine starke Aufhellung gewünscht, so ist es erfindungsgemäß bevorzugt, wenn zusätzlich eine Blondierzubereitung (C), enthaltend mindestens einen weiteren Bleichkraftverstärker, der Mischung aus Oxidationsmittelzubereitung (B) und der Zubereitung (A), enthaltend mindestens ein Oxidationsfarbstoffvorprodukt und mindestens ein Acylpyridinium-Derivat gemäß Formel (I), beigemischt wird.

Es kann dabei unerheblich sein, ob zunächst eine Mischung aus (A) und (B) hergestellt wird, und anschließend die Blondierzubereitung (C) zugemischt wird, oder ob eine davon verschiedene Reihenfolge der Vermischung der einzelnen Komponenten genutzt wird. Es ist bevorzugt, die einzelnen Zubereitungen in möglichst naher zeitlicher Abfolge zu vermischen und das anwendungsbereite Mittel vorzugsweise zeitnah auf die keratinischen Fasern zu applizieren.

Eine weitere Ausführungsform der vorliegenden Anmeldung ist daher ein Mittel zum Bleichen und Färben keratinischer Fasern, dadurch gekennzeichnet, dass es durch Vermischen von mindestens einer Oxidationsmittelszubereitung (B), enthaltend mindestens ein Oxidationsmittel, ausgewählt aus Wasserstoffperoxid dessen Anlagerungsverbindungen an feste Träger, mindestens einer Blondierzubereitung (C), enthaltend mindestens einen Bleichkraftverstärker, und mindestens einer Zubereitung (A), enthaltend in einem kosmetischen Träger mindestens ein Oxidationsfarbstoffvorprodukt und mindestens ein kationisches Acylpyridinium-Derivat gemäß Formel (I), vor der Anwendung hergestellt wird.

Für die starke Aufhellung sehr dunklen Haares ist der alleinige Einsatz von Wasserstoffperoxid oder dessen Anlagerungsprodukten an organische beziehungsweise anorganische Verbindungen oftmals nicht ausreichend. Die erfindungsgemäßen Mittel können daher zusätzlich noch weitere Blondier- und/oder Bleichmittel enthalten.

In solchen Fällen kann eine Kombination aus Wasserstoffperoxid und Persulfaten bzw. Peroxodisulfaten eingesetzt werden, woraus eine Steigerung des Aufhellvermögens der Mittel resultiert.

Daher kann es, sollte der Verbraucher den Wunsch nach einer sehr starken Blondierung verspüren, in einer weiteren Ausführungsform bevorzugt sein, wenn das erfindungsgemäße Färbemittel zusätzlich als Blondierzubereitung (C) mindestens eine anorganische Peroxoverbindung in dem Mittel zum Aufhellen der keratinischen Fasern enthält. Vorzugsweise ist die anorganische Peroxoverbindung ausgewählt aus Ammoniumpersulfat, Alkalimetallpersulfaten, Ammoniumperoxomonosulfat, Alkalimetallhydrogenperoxomonosulfaten, Alkalimetallperoxodiphosphaten und Erdalkalimetallperoxiden. Besonders bevorzugte anorganische Peroxoverbindung als Bleichkraftverstärker sind Ammoniumperoxodisulfat, Kaliumperoxodisulfat, Natriumperoxodisulfat, Kaliumhydrogenperoxomonosulfat, Kaliumperoxodiphosphat, Magnesiumperoxid und Bariumperoxid. Bevorzugte anorganische Peroxoverbindungen sind Peroxodisulfatsalze, insbesondere Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Es kann jedoch erfindungsgemäß vorteilhaft sein, wenn die Mittel frei von anorganischen Peroxoverbindungen sind. Die erfindungsgemäßen Mittel können jedoch anstelle und/oder zusätzlich zu den festen Peroxoverbindungen einen weiteren Bleichkraftverstärker enthalten.

Als zusätzliche Bleichkraftverstärker können im Rahmen dieser Erfindung Verbindungen, die unter Perhydrolysebedingungen aliphatische Peroxocarbonsäuren und/oder substituierte Perbenzoesäure ergeben, Kohlensäurederivate, Alkylcarbonate und -carbamate sowie Silylcarbonate und -carbamate eingesetzt werden. Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein. Die neben oder anstelle von Peroxoverbindungen eingesetzten Bleichkraftverstärker sind in den erfindungsgemäßen, kosmetischen Mitteln bevorzugt in Mengen von 0,05 bis 10 Gew.-%, insbesondere in Mengen von 0,2 bis 5 Gew.-%, jeweils bezogen auf das Gesamtgewicht des anwendungsbereiten Mittels, enthalten.

Obwohl prinzipiell keine Einschränkungen hinsichtlich der Formulierung der Blondierzubereitung (C) bestehen, hat es sich erfindungsgemäß als bevorzugt erwiesen, wenn die Zubereitung (C) wasserfrei formuliert ist.

Wasserfrei im Sinne der vorliegenden Erfindung bedeutet einen Wassergehalt bezogen auf die Zubereitung (C) von weniger als 5 Gew.-%, insbesondere von weniger als 2 Gew.-%. Blondierzubereitungen, die weniger als 0,1 Gew.-% Wasser, enthalten können erfindungsgemäß ganz besonders bevorzugt sein. Die Zubereitung (C) ist vorzugsweise als Pulver oder als wasserfreie Paste formuliert. Im Falle der Formulierung als wasserfreie Paste hat es sich als besonders bevorzugt erwiesen, wenn die Zubereitung (C) mindestens einen nicht hydroxylierten Fettsäureester mit einem Schmelzpunkt von höchstens 50°C, insbesondere von höchstens 30°C, und/oder mindestens eine $C_{10}$-$C_{30}$-Fettsäure mit mindestens einer zusätzlichen Hydroxygruppe und/oder ein Derivat davon, enthält. Ester von nicht hydroxylierten $C_6$-$C_{30}$-Alkylmonocarbonäuren mit $C_2$-$C_{30}$-Monoalkoholen eignen sich erfindungsgemäß bevorzugt als Fettsäureester. Bevorzugt sind die Monoester der Fettsäuren mit Monoalkoholen mit 2 bis 24 C-Atomen. Erfindungsgemäß besonders bevorzugt sind Isopropylmyristat, Isononansäure-C16-18-alkylester, 2-Ethylhexylpalmitat, Stearinsäure-2-ethylhexylester , Cetyloleat, Kokosfettalkohol-caprinat/-caprylat, n-Butylstearat, Oleylerucat, Isopropylpalmitat, Oleyloleat, Laurinsäurehexylester, Myristylmyristat , Cetearylisononanoat, Ölsäuredecylester.

Eine erfindungsgemäß bevorzugte Ausführungsform der vorliegenden Erfindung besteht darin, dass das anwendungsbereite Mittel einen pH- zwischen 7 und 11, insbesondere zwischen 8 und 10,5, insbesondere bevorzugt zwischen 8,5 und 10,0, besitzt. Bei den pH-Werten im Sinne der vorliegenden Erfindung handelt es sich um pH-Werte, die bei einer Temperatur von 22 °C gemessen wurden.

Üblicherweise wird der pH-Wert mit pH-Stellmitteln eingestellt. Zur Einstellung des pH-Werts sind dem Fachmann in der Kosmetik gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen, und Ammoniak. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Insbesondere bevorzugte Alkanolamine sind Monoethanolamin und Triethanolamin. Bevorzugt werden die Alkalisierungsmittel in einer Menge von 0,05 bis 10 Gew.%, insbesondere von 0,5 bis 5 Gew.-%, jeweils bezogen auf das Gesamtgewicht des anwendungsbereiten Mittels, enthalten.

Die Darreichungsform des erfindungsgemäßen Mittels ist eine Verpackungseinheit (Kit-of-Parts), welche in getrennt voneinander konfektionierten Containern

- mindestens eine Oxidationsmittelzubereitung (B), enthaltend mindestens ein Oxidationsmittel, und
- mindestens eine Zubereitung (A) enthält, wobei die Zubereitung (A) in einem kosmetischen Träger mindestens ein Oxidationsfarbstoffvorprodukt sowie mindestens ein Acylpyridinium-Derivat gemäß der Formel (I) enthält.

Wird ein besonders starker Aufhelleffekt gewünscht, so ist eine bevorzugte weitere Darreichungsform des erfindungsgemäßen Mittels eine Verpackungseinheit (Kit-of-Parts), welche in getrennt voneinander konfektionierten Containern

- mindestens eine Oxidationsmittelzubereitung (B), enthaltend mindestens ein Oxidationsmittel,
- mindestens eine Blondierzubereitung (C), enthaltend mindestens einen Bleichkraftverstärker, und
- mindestens eine Zubereitung (A) enthält, wobei die Zubereitung (A) in einem kosmetischen Träger mindestens ein Oxidationsfarbstoffvorprodukt sowie mindestens ein Acylpyridinium-Derivat gemäß der Formel (I) enthält.

Bevorzugt enthält die Mehrkomponentenverpackungseinheit (Kit-of-Parts) zusätzlich eine Gebrauchsanleitung. Darüber hinaus kann es bevorzugt sein, wenn weiterhin eine Applikationshilfe, wie beispielsweise ein Kamm oder ein Pinsel, und/oder eine persönliche Schutzausrüstung, wie beispielsweise Einweg-Handschuhe dem Kit beigefügt ist. Bezüglich weiterer bevorzugter Ausführungsformen der Mehrkomponentenverpackungseinheit (Kit-of-Parts) gilt mutatis mutandis das zu den erfindungsgemäßen Mitteln Gesagte.

Die Mischung der Zubereitungen (A), mit (B) oder gegebenenfalls der Zubereitungen (A), mit (B) und (C) vor der Anwendung führt zu einer erfindungsgemäßen Anwendungsmischung.

Das eigentliche Haarfärbemittel wird zweckmäßigerweise unmittelbar vor der Anwendung durch Mischung der Zubereitungen (A), mit (B) sowie gegebenenfalls (C) hergestellt. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Ein weiterer Gegenstand der vorliegenden Erfindung ist daher ein Verfahren zur Färben und Aufhellen menschlicher Haare, bei dem ein erfindungsgemäßes Mittel gemäß obiger Vorgaben auf das Haar aufgetragen wird, für eine Einwirkzeit von 2 bis 45 Minuten, bevorzugt von 5 bis 20 Minuten, ganz besonders bevorzugt von 8 bis 15 Minuten, auf dem Haar belassen wird, und aus dem Haar ausgespült oder mit einem Shampoo ausgewaschen wird.

Ein weiterer Gegenstand der Erfindung ist die Verwendung eines erfindungsgemäßen Mittels zum Färben und gleichzeitigem Aufhellen von keratinhalten Fasern, insbesondere menschlichen Haaren. Bezüglich weiterer bevorzugter Ausführungsformen der erfindungsgemäßen Verfahren und Verwendungen gilt mutatis mutandis das zu den erfindungsgemäßen Mitteln Gesagte.

Die nachfolgenden Beispiele sollen bevorzugte Ausführungsformen der Erfindung erläutern, ohne sie jedoch einzuschränken.

## Beispiele

### 1. Synthesebeispiele

1.1. *Synthese von 4-Acetyl-1-methylpyridinium-p-toluolsulfonat*

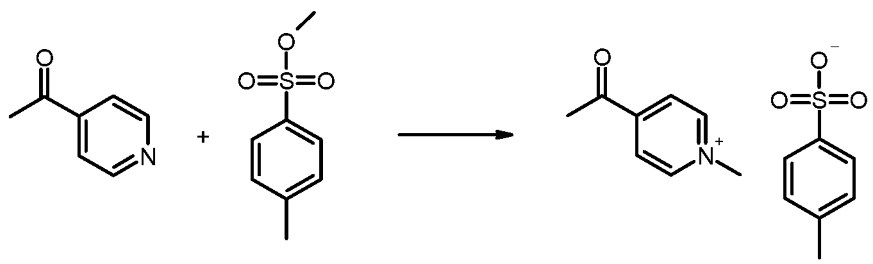

Es wurden 30,0 g (0,25 mol) 4-Acetylpyridin und 55,8 g (0,30 mol) p-Toluolsulfonsäuremethylester in 500 ml

Ethanol für 5 Stunden unter Rückfluss erhitzt. Das Lösungsmittel wurde im Vakuum am Rotationsverdampfer abgezogen und der Rückstand mit Ether digeriert. Nach Abtrennen der Etherphase kristallisierte das Produkt nach und nach aus. Das Produkt wurde im Vakuum getrocknet. Ausbeute: 59,9 g (82,5 %); $^1$H-NMR (400 MHz, DMSO-$d_6$): δ [ppm] = 2,26 (s, 3H); 2,72 (s, 3H); 3,39 (s, 3H); 7,11 (d, 2H); 7,49 (d, 2H); 8,42 (d, 2H); 9,20 (d, 2H); $^{13}$C-NMR (400 MHz, DMSO-d6): δ [ppm] = 20,8; 26,4; 48,1; 124,8; 125,3; 127,7; 138,9; 145,2; 146,5; 148,3; 195,8.

## 2. Beispiele zur Blondierung

2.1 *Herstellung von Blondiercremes*

Aus den nachfolgend aufgelisteten Bestandteilen wurden Blondiercremes hergestellt:

| **Rohstoff** | **Gew.-%** | |
|---|---|---|
| | **V1** | **E1** |
| Hydrenol D | 4,0 | 4,0 |
| Lorol techn. | 2,4 | 2,4 |
| Eumulgin B3 | 0,2 | 0,2 |
| Eumulgin B2 | 0,2 | 0,2 |
| Mergital CS 50 A | 0,8 | 0,8 |
| 1,2-Propandiol | 0,4 | 0,4 |
| Xanthan FN | 0,1 | 0,1 |
| Crodafos CES | 2,0 | 2,0 |
| Amphoterge K-2 | 2,0 | 2,0 |
| Merquat 281 | 3,0 | 3,0 |
| Ammoniumsulfat | 2,0 | 2,0 |
| Natriumsulfit | 0,1 | 0,1 |
| L-Arginin | 1,0 | 1,0 |
| p-Toluylendiaminsulfat | 0,1 | 0,1 |
| Resorcin | 0,1 | 0,1 |
| HEDP 60 % | 0,2 | 0,2 |
| Natriumsilikat 40/42 | 0,5 | 0,5 |
| Ammoniak 25 % | 10,0 | 10,0 |
| ***4-Acetyl-1-methylpyridinium-p-toluolsulfonat gemäß 1.1*** | **---** | **1,0** |
| Wasser | ad 100 | ad 100 |

*2.2 Vermischen mit der Entwicklerdispersion*

Jede Blondiercreme wurde im Verhältnis 1:1 mit einer wie folgt zusammengesetzten Entwicklerdispersion ausgemischt. Der pH-Wert der Anwendungsmischung lag zwischen 9 und 10,2.

| **Rohstoff** | **Gew.-%** |
|---|---|
| Natronlauge 45 % | 0,73 |
| Dipicolinsäure | 0,10 |
| Dinatriumpyrophosphat | 0,03 |
| HEDP 60 % | 1,5 |

(fortgesetzt)

| Rohstoff | Gew.-% |
|---|---|
| FAEOS-Na C12-14 2EO 27% | 2,0 |
| Dow Corning DB 110 A | 0,07 |
| Aculyn 33A | 2,5 |
| Wasserstoffperoxid 50 % | 20,00 |
| Wasser | ad 100 |

| | |
|---|---|
| Hydrenol® D | INCI-Bezeichnung: Cetearyl Alcohol (Cognis) |
| Lorol® C12-18, techn. | INCI-Bezeichnung: Coconut Alcohol (Cognis) |
| Eumulgin® B3 | INCI-Bezeichnung: Ceteareth-30 (Cognis) |
| Eumulgin® B 2 | INCI-Bezeichnung: Ceteareth-20 (Cognis) |
| Mergital® CS 50 | INCI-Bezeichnung: Ceteareth-50 (Cognis) |
| Crodafos® CES | INCI-Bezeichnung: Cetearyl Alcohol, Dicetyl Phosphate, Ceteth-10 Phosphate (Croda) |
| Merquat® 281 | INCI-Bezeichnung: Polyquaternium-22 (Nalco) |
| Amphoterge® K-2 | INCI-Bezeichnung: Disodium Cocoamphodipropionate (Lonza) |
| Dow Corning® DB 110 A | INCI-Bezeichnung: Dimethicone (Dow Corning) |
| Aculyn® 33A | INCI-Bezeichnung: Acrylates Copolymer (ISP) |

Für den Färbeprozess wurde auf Strähnen dunkelblonden Haares (Code Kerling 7/0) von ca. 0,7 g Gewicht die 4-fache Menge der fertigen Anwendungsmischung appliziert. Nachdem die Strähnen für 10 Minuten bei 32 °C blondiert wurden, wurden sie mit einem handelsüblichen Shampoo gewaschen und mit einem Föhn getrocknet.

*2.3 Auswertung der Aufhell- und Färbeleistung*

Jede Haarsträhne wurde vor und nach dem Bleichvorgang farbmetrisch vermessen. Bei jeder Messung wurde ein L-Wert, a-Wert und ein b-Wert ermittelt. Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben.

Als Maß für die Aufhellleistung der jeweiligen Rezeptur wurde der dL-Wert gemäß folgender Gleichung (I) herangezogen:

$$\Delta L = L_{nachher} - L_{vorher} \qquad \text{Gleichung (I)}$$

$L_{nachher}$ = Helligkeit der Strähne nach dem Färbevorgang
$L_{vorher}$ = Helligkeit der Strähne vor dem Färbevorgang

Farbunterschiede wurden mittels des ΔE-Wertes charakterisiert, der aus den drei ermittelten Werten entsprechend Gleichung (II) berechnet wird:

$$\Delta E = \sqrt{(\Delta L)^2 + (\Delta a)^2 + (\Delta b)^2} \qquad \text{Gleichung (II)}$$

$\Delta a = a_{nachher} - a_{vorher}$ mit $a_{nachher}$ = a-Wert nach dem Färbevorgang
$a_{vorher}$ = a-Wert vor dem Färbevorgang

$\Delta b = b_{nachher} - b_{vorher}$ mit $b_{nachher}$ = b-Wert nach dem Färbevorgang
$b_{vorher}$ = b-Wert vor dem Färbevorgang

Für jede Rezeptur erfolgte eine Doppelbestimmung, aus den Einzelwerten wurde jeweils der Mittelwert gebildet. Je größer der ΔL-Wert ist, desto besser ist die Aufhellleistung der jeweiligen Rezeptur. Je größer der ΔE-Wert ist, desto größer ist der Farbunterschied im Vergleich zur Ausgangshaarfarbe.

*2.4. Ergebnisse der Aufhell- und Färbeleistung*

**Aufhell- und Färbeleistung bei dunkelblonden Strähnen (Kerling 7/0)**

| ΔL (Rezeptur V1) | ΔL (Rezeptur **E1**) |
|---|---|
| 3,82 | **5,68** |
| | |
| ΔE (Rezeptur V1) | ΔE (Rezeptur **E1**) |
| 5,80 | **8,42** |

Bei einem Vergleich der farbmetrischen Messwerte ist eindeutig ersichtlich, dass mit den erfindungsgemäßen Rezepturen größere ΔL-Werte erzielt werden können. Durch Anwendung dieser Rezepturen werden damit die Blondierleistungen verbessert, wodurch sie bezogen auf den Stand der Technik als überlegen einzustufen sind. Desgleichen werden bei den erfindungsgemäßen Rezepturen größere ΔE-Werte als bei den Vergleichsrezepturen erhalten. Bezogen auf die Ausgangshaarfarbe kann somit durch die in dieser Erfindung beschriebene Zusammensetzung bei einer Applikationszeit von nur 10 Minuten ein größerer Farbunterschied erzeugt werden.

## Patentansprüche

1. Mittel zum Färben und gleichzeitigem Aufhellen von keratinischen Fasern, **dadurch gekennzeichnet, dass** es unmittelbar vor der Anwendung durch Vermischen mindestens zweier Zubereitungen hergestellt wird, wobei die mindestens zwei Zubereitungen in mindestens zwei getrennt konfektionierten Containern bereitgestellt werden, und wobei ein Container ein Färbemittel (A), welches in einem kosmetischen Träger mindestens ein Oxidationsfarbstoffvorprodukt sowie mindestens ein Acylpyridinium-Derivat gemäß der Formel (I) enthält,

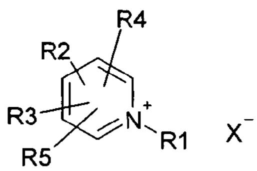

(I),

worin

R1 für eine $C_1$-$C_6$-Alkylgruppe, eine $C_2$-$C_6$-Alkenylgruppe, eine $C_2$-$C_6$-Hydroxy-alkylgruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe steht,
R2 für eine Acylgruppe R'C(O) steht, in der R' für eine $C_1$-$C_6$-Alkylgruppe steht,
R3, R4 und R5 unabhängig voneinander ein Wasserstoffatom, eine Hydroxygruppe, eine Aminogruppe, eine Di-($C_1$-$C_6$-alkyl)aminogruppe, eine $C_1$-$C_6$-Alkoxygruppe, eine $C_1$-$C_6$-Alkoxy-$C_2$-$C_6$-alkylgruppe, ein Halogen, eine Nitrogruppe, eine Carboxygruppe, eine Nitrilgruppe, eine gegebenenfalls substituierte Arylgruppe, eine $C_1$-$C_6$-Alkylgruppe, eine $C_2$-$C_6$-Alkenylgruppe, eine gegebenenfalls substituierte Heteroarylgruppe darstellen können, und

das Anion $X^-$ für ein physiologisch verträgliches Anion steht.
und ein weiterer Container eine Oxidationsmittelzubereitung (B), enthaltend mindestens ein Oxidationsmittel, enthält.

2. Mittel nach Anspruch 1, **dadurch gekennzeichnet, dass** der Rest R1 gemäß Formel (I) für eine $C_1$-$C_6$-Alkylgruppe, für eine $C_2$-$C_6$-Alkenylgruppe oder für eine $C_2$-$C_6$-Hydroxyalkylgruppe steht.

3. Mittel nach den Ansprüchen 1 und 2, **dadurch gekennzeichnet, dass** der Rest R2 gemäß Formel (I) für eine Acylgruppe R'C(O) steht, in der R' für eine Methylgruppe, steht.

4. Mittel nach den Ansprüchen 1 bis 3, **dadurch gekennzeichnet, dass** der Rest R2 gemäß Formel (I) in der 2-Position oder in der 4-Position steht.

5. Mittel nach den Ansprüchen 1 bis 4, **dadurch gekennzeichnet, dass** die Reste R3, R4 und R5 gemäß Formel (I) ein Wasserstoffatom darstellen.

6. Mittel nach einem der Ansprüche 1 bis 5, **dadurch gekennzeichnet, dass** das physiologisch verträgliche Anion X- für ein Halogenidion (insbesondere Chlorid oder Bromid), Hydrogensulfat, ½ Sulfat, p-Toluolsulfonat, Benzolsulfonat oder Acetat steht.

7. Mittel nach einem der Ansprüche 1 bis 6, **dadurch gekennzeichnet, dass** mindestens ein Acylpyridinium-Derivat gemäß Formel (I) ausgewählt aus Verbindungen der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methylpyridiniumchlorid, 4-Acetyl-1-methylpyridiniumhydrogensulfat, 4-Acetyl-1-methylpyridiniumacetat, 4-Acetyl-1-allylpyridinium-p-toluölsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumbromid, 2-Acetyl-1-allylpyridiniumchlorid, 2-Acetyl-1-allylpyridiniumhydrogensulfat oder 2-Acetyl-1-allylpyridiniumacetat, enthalten ist.

8. Mittel nach einem der Ansprüche 1 bis 7, **dadurch gekennzeichnet, dass** die Acylpyridinium-Derivate gemäß Formel (I) zu 0,01 bis 15 Gew.-%, bevorzugt zu 0,1 bis 12 Gew.% und ganz besonders bevorzugt zu 0,5 bis 5 Gew.-%, jeweils bezogen auf das Gesamtgewicht des anwendungsbereiten Mittels, enthalten sind.

9. Mittel nach einem der Ansprüche 1 bis 8" **dadurch gekennzeichnet, dass** die Oxidationsmittelzubereitung (B) als Oxidationsmittel Wasserstoffperoxid und/oder eines seiner festen Anlagerungsprodukte an organische oder anorganische Verbindungen enthält.

10. Mittel nach einem der Ansprüche 1 bis 8" **dadurch gekennzeichnet, dass** es das Oxidationsmittel - bezogen auf das Gesamtgewicht des anwendungsbereiten Mittels- zu 0,5 bis 12 Gew.-%, vorzugsweise zu 2 bis 10 Gew.% und insbesondere bevorzugt zu 3 bis 6 Gew.-% (berechnet als 100 %-iges $H_2O_2$) enthält.

11. Mittel nach einem der Ansprüche 1 bis 10, **dadurch gekennzeichnet, dass** es zusätzlich einen toxikologisch unbedenklichen Co-Bleichaktivator gemäß Formel (II) und/oder dessen physiologisch verträgliches Salz enthält,

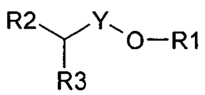

(II)

worin

Y für eine Carbonyl-Gruppe, eine direkte Bindung oder Methylen-Gruppe steht,
R1 für Wasserstoff, eine $C_1$-$C_4$-Alkylgruppe, ein physiologisch verträgliches Kation oder eine $SO_3^-$ oder eine $PO_3^{2-}$- Gruppe steht,
R2 für eine Amino-, eine Methylamino-, eine Dimethylamino-, eine Trimethylammonio-Gruppe, Phenyl, Benzyl, Phenoxymethyl, 1-Naphthyl, 2-Naphthyl, 2-, 3-, 4-Toluolyl, oder eine R4-O-$(CH_2CH_2O)_n$-Gruppe steht, worin R4 für eine $C_6$-$C_{20}$-Alkylgruppe und n für eine Zahl von größer als 15 steht,
R3 für Wasserstoff oder eine gegebenenfalls verzweigte $C_1$-$C_6$-Alkylgruppe steht,

mit der Maßgabe, dass,

wenn Y für eine Carbonyl-Gruppe steht,
R1 für Wasserstoff, eine $C_1$-$C_4$-Alkylgruppe oder ein physiologisch verträgliches Kation steht,
R2 für eine Amino-, eine Methylamino-, eine Dimethylamino- oder eine Trimethylammonio-Gruppe steht, und
R3 für Wasserstoff oder eine gegebenenfalls verzweigte $C_1$-$C_6$-Alkylgruppe steht, dass,
wenn Y für eine direkte Bindung steht,
R1 für Wasserstoff und
R2 für Phenyl, Benzyl, Phenoxymethyl, 1-Naphthyl, 2-Naphthyl, 2-, 3- oder 4-Toluolyl steht, und
R3 für Wasserstoff oder eine gegebenenfalls verzweigte $C_1$-$C_6$-Alkylgruppe steht, und dass,
wenn Y für eine Methylen-Gruppe steht,
R1 für eine $SO_3^-$- oder eine $PO_3^{2-}$-Gruppe steht,
R2 für eine R4-$O(CH_2CH_2O)_n$-Gruppe steht, worin R4 für eine $C_6$-$C_{20}$-Alkylgruppe und n für eine Zahl von größer als 15 steht, und
R3 für Wasserstoff steht.

**12.** Mittel nach einem der Ansprüche 1 bis 11, **dadurch gekennzeichnet**, das der pH-Wert der verwendungsbereiten Anwendungsmischung zwischen 7 und 11, insbesondere zwischen 8 und 10,5, liegt.

**13.** Verfahren zum Färben und gleichzeitigen Aufhellen von keratinischen Fasern, **dadurch gekennzeichnet, dass** ein Mittel nach einem der Ansprüche 1 bis 12 auf die keratinhaltigen Fasern aufgebracht, 5 bis 45 Minuten, bevorzugt 5 bis 20 Minuten, auf der Faser belassen und anschließend wieder ausgespült oder mit einem Shampoo ausgewaschen wird.

**14.** Kosmetische Verwendung eines Mittels nach einem der Ansprüche 1 bis 12 zum Färben und gleichzeitigem Aufhellen von keratinhalten Fasern, insbesondere menschlichen Haaren.

**Claims**

**1.** An agent for dyeing and simultaneously lightening keratinic fibers; **characterized in that** it is produced directly prior to use by blending at least two preparations, wherein the at least two preparations are provided in at least two separately packaged containers, and wherein one container comprises a dye (A) that comprises in a cosmetic carrier at least one oxidation dye precursor as well as at least one acylpyridinium derivative according to Formula (I),

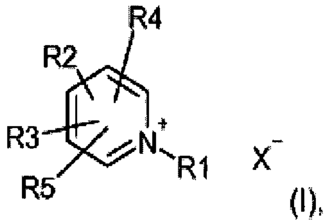

(I),

in which

R1 stands for a $C_1$-$C_6$ alkyl group, a $C_2$-$C_6$ alkenyl group, a $C_2$-$C_6$ hydroxyalkyl group, a $C_1$-$C_6$ alkoxy $C_2$-$C_6$ alkyl group, a carboxy $C_2$-$C_6$ alkyl group, an aryl $C_1$-$C_6$ alkyl group, a heteroaryl $C_1$-$C_6$ alkyl group, an aryl group or a heteroaryl group,
R2 stands for an acyl group R'C(O), in which R' stands for a $C_1$-$C_6$ alkyl group,
R3, R4 and R5 independently of one another can represent a hydrogen atom, a hydroxyl group, an amino group, a di-($C_1$-$C_6$ alkyl)amino group, a $C_1$-$C_6$, alkoxy group, a $C_1$-$C_6$ alkoxy $C_2$-$C_6$ alkyl group, a halogen, a nitro group, a carboxy group, a nitrile group, an optionally substituted aryl group, a $C_1$-$C_6$ alkyl group, a $C_2$-$C_6$ alkenyl group, an optionally substituted heteroaryl group, and

the anion X- stands for a physiologically acceptable anion,
and an additional container, comprising at least one oxidizing agent, comprises an oxidizing agent preparation (B).

2. The agent according to claim 1, **characterized in that** the group R1 according to Formula (I) stands for a $C_1$-$C_6$ alkyl group, for a $C_2$-$C_6$ alkenyl group or for a $C_2$-$C_6$ hydroxyalkyl group.

3. The agent according to claims 1 and 2, **characterized in that** the group R2 according to Formula (I) stands for an acyl group R'C(O), in which R' stands for a methyl group.

4. The agent according to claims 1 to 3, **characterized in that** the group R2 according to Formula (I) stands in the 2-position or in the 4-position.

5. The agent according to claims 1 to 4, **characterized in that** the groups R3, R4 and R5 according to Formula (I) represent a hydrogen atom.

6. The agent according to one of claims 1 to 5, **characterized in that** the physiologically acceptable anion X- stands for a halide ion (especially chloride or bromide), hydrogen sulfate, ½ sulfate, p-toluene sulfonate, benzene sulfonate or acetate.

7. The agent according to one of claims 1 to 6, **characterized in that** it comprises at least one acylpyridinium derivative according to Formula (I) selected from compounds of the group consisting of 4-acetyl-1-methylpyridinium p-toluene sulfonate, 4-acetyl-1-methylpyridinium benzene sulfonate, 4-acetyl-l-methylpyridinium bromide, 4-acetyl-1-methylpyridinium chloride, 4-acetyl-1-methylpyridinium hydrogen sulfate, 4-acetyl-l-methylpyridinium acetate, 4-acetyl-1-allylpyridinium p-toluene sulfonate, 4-acetyl-l-allylpyridinium benzene sulfonate, 4-acetyl-1-allylpyridinium bromide, 4-acetyl-l-allylpyridinium chloride, 4-acetyl-1-allylpyridinium hydrogen sulfate, 4-acetyl-1-allylpyridinium acetate, 2-acetyl-1-methylpyridinium p-toluene sulfonate, 2-acetyl-1-methylpyridinium benzene sulfonate, 2-acetyl-1-methylpyridinium bromide, 2-acetyl-1-methylpyridinium chloride, 2-acetyl-1-methylpyridinium hydrogen sulfate, 2-acetyl-1-methylpyridinium acetate, 2-acetyl-l-allylpyridinium p-toluene sulfonate, 2-acetyl-1-allylpyridinium benzene sulfonate, 2-acetyl-l-allylpyridinium bromide, 2-acetyl-1-allylpyridinium chloride, 2-acetyl-1-allylpyridinium hydrogen sulfate or 2-acetyl-1-allylpyridinium acetate.

8. The agent according to one of claims 1 to 7, **characterized in that** it comprises the acylpyridinium derivatives according to Formula (I) in 0.01 to 15 wt.%, preferably 0.1 to 12 wt.% and quite particularly preferably 0.5 to 5 wt.%, based on the total weight of the ready-to-use agent.

9. The agent according to one of claims 1 to 8, **characterized in that** the oxidizing agent preparation (B) comprises hydrogen peroxide and/or one of its solid addition products on organic or inorganic compounds as the oxidizing agent.

10. The agent according to one of claims 1 to 8, **characterized in that** it comprises, based on the total weight of the ready-to-use composition, 0.5 to 12 wt.%, preferably 2 to 10 wt.% and particularly preferably 3 to 6 wt.% of the oxidizing agent (calculated as $H_2O_2$ 100 %).

11. The agent according to one of claims 1 to 10, **characterized in that** it additionally comprises a toxicologically acceptable bleach co-activator according to Formula (II) and/or a physiologically acceptable salt thereof,

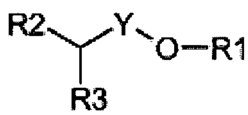

(II)

in which

Y stands for a carbonyl group, a direct bond or methylene group,
R1 stands for hydrogen, a $C_1$-$C_4$ alkyl group, a physiologically acceptable cation or a $SO_3^-$ or a $PO_3^{2-}$ group,
R2 stands for an amino, a methylamino, a dimethylamino, a trimethylammonio group, phenyl, benzyl, phenoxymethyl, 1-naphthyl, 2-naphthyl, 2-, 3-, 4-toluyl, or a R4-O-$(CH_2CH_2O)_n$ group, in which R4 stands for a $C_6$-$C_{20}$ alkyl group and n stands for a number greater than 15,
R3 stands for hydrogen or an optionally branched $C_1$-$C_6$ alkyl group,

with the proviso that,

when Y stands for a carbonyl group,
R1 stands for hydrogen, a $C_1$-$C_4$ alkyl group or a physiologically acceptable cation,
R2 stands for an amino, a methylamino, a dimethylamino or a trimethylammonio group, and
R3 stands for hydrogen or an optionally branched $C_1$-$C_6$ alkyl group, that,
when Y stands for a direct bond,
R1 stands for hydrogen and
R2 stands for phenyl, benzyl, phenoxymethyl, 1-naphthyl, 2-naphthyl, 2-, 3- or 4-toluyl, and
R3 stands for hydrogen or an optionally branched $C_1$-$C_6$ alkyl group,
and that,
when Y stands for a methylene group,
R1 stands for a $SO_3^-$ or a $PO_32^-$ group,
R2 stands for a R4-O-$(CH_2CH_2O)_n$ group, in which R4 stands for a $C_6$-$C_{20}$ alkyl group and n stands for a number greater than 15, and
R3 stands for hydrogen.

12. The agent according to one of claims 1 to 11, **characterized in that** the pH of the ready-to-use application mixture is between 7 and 11, especially between 8 and 10.5.

13. A method for dyeing and simultaneously lightening keratinic fibers, **characterized in that** an agent according to one of claims 1 to 12 is applied onto the keratinic fibers, left on the fibers for 5 to 45 minutes, preferably 5 to 20 minutes, and then rinsed out again or washed out with a shampoo.

14. Cosmetic use of an agent according to one of claims 1 to 12 for dyeing and simultaneously lightening keratinic fibers, especially human hair.

**Revendications**

1. Agent pour la teinture et la décoloration simultanée de fibres kératiniques, **caractérisé en ce qu'**on le prépare directement avant l'utilisation par mélange d'au moins deux préparations, lesdites au moins deux préparations étant fournies dans au moins deux récipients confectionnés de manière séparée, et un récipient contenant un colorant (A) qui contient dans un support cosmétique au moins un précurseur de colorant d'oxydation et au moins un dérivé d'acylpyridinium répondant à la formule (I)

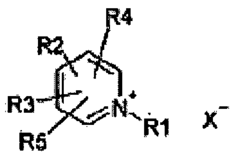

(I),

dans laquelle

R1 représente un groupe alkyle en $C_1$-$C_6$, un groupe alcényle en $C_2$-$C_6$, un groupe hydroxyalkyle en $C_2$-$C_6$, un groupe alcoxy(en $C_1$-$C_6$)alkyle en $C_2$-$C_6$, un groupe carboxyalkyle en $C_2$-$C_6$, un groupe arylalkyle en $C_1$-$C_6$, un groupe hétéroarylalkyle en $C_1$-$C_6$, un groupe aryle ou un groupe hétéroaryle ;
R2 représente un groupe acyle R'C(O) dans lequel R' représente un groupe alkyle en $C_1$-$C_6$;
R3, R4 et R5 peuvent représenter, indépendamment l'un de l'autre, un atome d'hydrogène, un groupe hydroxyle, un groupe amino, un groupe di(alkyl en $C_1$-$C_6$)amino, un groupe alcoxy en $C_1$-$C_6$, un groupe alcoxy(en $C_1$-$C_6$) alkyle en $C_2$-$C_6$, un atome d'halogène, un groupe nitro, un groupe carboxyle, un groupe nitrile, un groupe aryle facultativement substitué, un groupe alkyle en $C_1$-$C_6$, un groupe alcényle en $C_2$-$C_6$, un groupe hétéroaryle facultativement substitué ; et

l'anion X- représente un anion physiologiquement acceptable ;
et un autre récipient contient une préparation d'agent d'oxydation (B) contenant au moins un agent d'oxydation.

2. Agent selon la revendication 1, **caractérisé en ce que** le résidu R1 répondant à la formule (I) représente un groupe

alkyle en $C_1$-$C_6$, un groupe alcényle en $C_2$-$C_6$ ou un groupe hydroxyalkyle en $C_2$-$C_6$.

3. Agent selon les revendications 1 et 2, **caractérisé en ce que** le résidu R2 répondant à la formule (I) représente un groupe acyle R'C(O) dans lequel R' représente un groupe méthyle.

4. Agent selon les revendications 1 à 3, **caractérisé en ce que** le résidu R2 répondant à la formule (I) se trouve en position 2 ou en position 4.

5. Agent selon les revendications 1 à 4, **caractérisé en ce que** les résidus R3, R4 et R5 répondant à la formule (I) représentent un atome d'hydrogène.

6. Agent selon l'une quelconque des revendications 1 à 5, **caractérisé en ce que** l'anion physiologiquement acceptable X- représente un ion halogénure (en particulier, chlorure ou bromure), un hydrogénosulfate, un semi-sulfate, un p-toluènesulfonate, un benzènesulfonate ou un acétate.

7. Agent selon l'une quelconque des revendications 1 à 6, **caractérisé en ce qu'**il contient au moins un dérivé d'acylpyridinium répondant à la formule (I) choisi parmi des composés du groupe qui est formé par le p-toluènesulfonate de 4-acétyl-1-méthylpyridinium, le benzènesulfonate de 4-acétyl-1-méthylpyridinium, le bromure de 4-acétyl-1-méthylpyridinium, le chlorure de 4-acétyl-1-méthylpyridinium, l'hydrogénosulfate de 4-acétyl-1-méthylpyridinium, l'acétate de 4-acétyl-1-méthylpyridinium, le p-toluènesulfonate de 4-acétyl-1-allylpyridinium, le benzènesulfonate de 4-acétyl-1-allylpyridinium, le bromure de 4-acétyl-1-allylpyridinium, le chlorure de 4-acétyl-1-allylpyridinium, l'hydrogénosulfate de 4-acétyl-1-allylpyridinium, l'acétate de 4-acétyl-1-allylpyridinium, le p-toluènesulfonate de 2-acétyl-1-méthylpyridinium, le benzènesulfonate de 2-acétyl-1-méthylpyridinium, le bromure de 4-acétyl-1-méthylpyridinium, le chlorure de 2-acétyl-1-méthylpyridinium, l'hydrogénosulfate de 2-acétyl-1-méthylpyridinium, l'acétate de 2-acétyl-1-méthylpyridinium, le p-toluènesulfonate de 2-acétyl-1-allylpyridinium, le benzènesulfonate de 2-acétyl-1-allylpyridinium, le bromure de 2-acétyl-1-allylpyridinium, le chlorure de 2-acétyl-1-allylpyridinium, l'hydrogénosulfate de 2-acétyl-1-allylpyridinium ou l'acétate de 2-acétyl-1-allylpyridinium.

8. Agent selon l'une quelconque des revendications 1 à 7, **caractérisé en ce qu'**il contient les dérivés d'acylpyridinium répondant à la formule (I) à concurrence de 0,01 à 15 % en poids, de préférence à concurrence de 0,1 à 12 % en poids et de manière tout particulièrement préférée à concurrence de 0,5 à 5 % en poids, chaque fois rapportés au poids total de l'agent prêt à l'emploi.

9. Agent selon l'une quelconque des revendications 1 à 8, **caractérisé en ce que** la préparation d'agent d'oxydation (B) contient, à titre d'agent d'oxydation, du peroxyde hydrogène et/ou un de ses produits solides d'addition à des composés organiques ou inorganiques.

10. Agent selon l'une quelconque des revendications 1 à 8, **caractérisé en ce qu'**il contient l'agent d'oxydation - rapportés au poids total de l'agent prêt à l'emploi - à concurrence de 0,5 à 12 % en poids, de préférence à concurrence de 2 à 10 % en poids et de manière particulièrement préférée à concurrence de 3 à 6 % en poids (calculés sous la forme de $H_2O_2$ à 100 %).

11. Agent selon l'une quelconque des revendications 1 à 10, **caractérisé en ce qu'**il contient en outre un coactivateur du blanchiment acceptable du point de vue toxicologique répondant à la formule (II) et/ou son sel physiologiquement acceptable,

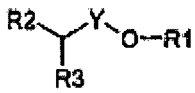

(II)

dans laquelle

Y représente un groupe carbonyle, une liaison directe ou un groupe méthylène ;
R1 représente un atome d'hydrogène, un groupe alkyle en $C_1$-$C_4$, un cation physiologiquement acceptable ou un groupe $SO_3^-$ ou encore un groupe $PO_3^{2-}$ ;
R2 représente un groupe amino, un groupe méthylamino, un groupe diméthylamino, un groupe triméthylammonio, un groupe phényle, un groupe benzyle, un groupe phénoxyméthyle, un groupe 1-naphtyle, un groupe

2-naphtyle, un groupe 2-, 3-, 4-toluolyle ou un groupe R4-O-$(CH_2CH_2O)_n$, dans lequel R4 représente un groupe alkyle en $C_6$-$C_{20}$, et n représente un nombre supérieur à 15 ;
R3 représente un atome d'hydrogène ou un groupe alkyle en $C_1$-$C_6$ facultativement ramifié ;

avec cette mesure que :

lorsque Y représente un groupe carbonyle :

R1 représente un atome d'hydrogène, un groupe alkyle en $C_1$-$C_4$ ou un cation physiologiquement acceptable ;
R2 représente un groupe amino, un groupe méthylamino, un groupe diméthylamino ou un groupe triméthylammonio ; et
R3 représente un atome d'hydrogène ou un groupe alkyle en $C_1$-$C_6$ facultativement ramifié ;

que :

lorsque Y représente une liaison directe :

R1 représente un atome d'hydrogène ; et
R2 représente un groupe phényle, un groupe benzyle, un groupe phénoxyméthyle, un groupe 1-naphtyle, un groupe 2-naphtyle, un groupe 2-, 3- ou 4-toluolyle ; et
R3 représente un atome d'hydrogène ou un groupe alkyle en $C_1$-$C_6$ facultativement ramifié ;

et que :

lorsque Y représente un groupe méthylène :

R1 représente un groupe $SO_3^-$ ou un groupe $PO_3^{2-}$;
R2 un groupe R4-O-$(CH_2CH_2O)_n$, dans lequel R4 représente un groupe alkyle en $C_6$-$C_{20}$, et n représente un nombre supérieur à 15 ; et
R3 représente un atome d'hydrogène.

**12.** Agent selon l'une quelconque des revendications 1 à 11, **caractérisé en ce que** la valeur de pH du mélange d'application prêt à l'emploi se situe entre 7 et 11, en particulier entre 8 et 10,5.

**13.** Procédé pour la teinture et la décoloration simultanée de fibres kératiniques, **caractérisé en ce qu'**on applique un agent selon l'une quelconque des revendications 1 à 12 sur les fibres kératiniques, on l'abandonne sur les fibres pendant un laps de temps de 5 à 45 minutes, de préférence de 5 à 20 minutes, et on l'en élimine ensuite par rinçage ou par lavage avec un shampooing.

**14.** Utilisation cosmétique d'un agent selon l'une quelconque des revendications 1 à 12, pour la teinture et la décoloration simultanée de fibres kératiniques, en particulier de cheveux humains.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102007047685 A1